# EUROPEAN PATENT APPLICATION

(11) **EP 1 619 207 A2**
(43) Date of publication of application: **25.01.2006**
(21) Application number: 05016493.8
(22) Date of filing: 01.09.2000
(51) Int. Cl.: C07K 16/00, C07H 21/04, A61K 39/00, C07K 14/02, C07K 14/03, C07K 14/08, C07K 14/11, C07K 14/16, C07K 14/18, C12N 9/90, C07K 7/06, A61K 39/29

(54) **HLA-A2.1 binding peptides derived from HCV and their uses**

(62) Divisional of application: 00964938.5
(71) Applicant: Epimmune Inc., San Diego, CA 92121 (US)
(72) Inventor: Grey, Howard M., La Jolla, CA 92037 (US); Sette, Alessandro, La Jolla, CA92037 (US); Sidney, John, La Jolla, CA92037 (US); Southwood, Scott, Santee, CA92071 (US)
(74) Representative: Thomson, Paul Anthony

(57) **Abstract**

An isolated HCV peptide of 15 residues or less, comprising a sequence selected from the group consisting of:
KLVALGINAV (SEQ ID NO:175)
SLMAFTAAV (SEQ ID NO:176)
CINGVCWTV (SEQ ID NO:177)
TISGVLWQV (SEQ ID NO:178)
SISGVLWQV (SEQ ID NO:179)
SLMAFTASV(SEQ ID NO:180)
GLRDCTMLV (SEQ ID NO:181)
KLVALGVNAV(SEQ ID NO:182)
KLSGLGLNAV (SEQ ID NO:183)
LLCPAGHAV (SEQ ID NO:186)
LLFNILGWV (SEQ ID NO:187) and
any of the above sequences modified by one or two conservative substitutions.

## Description

The present application is a continuation in part of USSN 08/589,108 and is related to the following patent applications, USSN 60/013,980, USSN 08/454,033 and USSN 08/349,177, which is a continuation in part of USSN 08/159,184, which is a continuation in part of USSN 08/073,205, which is a continuation in part of 08/027,146. It is also related to USSN 08/205,713 and USSN 08/753,622. All of which are incorporated herein by reference.

### BACKGROUND OF THE INVENTION

The present invention relates to compositions and methods for preventing, treating or diagnosing a number of pathological states such as viral diseases and cancers. In particular, it provides novel peptides capable of binding selected major histocompatibility complex (MHC) molecules and inducing an immune response.

MHC molecules are classified as either Class I or Class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs.

Investigations of the crystal structure of the human MHC class I molecule, HLA-A2.1, indicate that a peptide binding groove is created by the folding of the α1 and α2 domains of the class I heavy chain (Bjorkman et al., Nature 329:506(1987). In these investigations, however, the identity of peptides bound to the groove was not determined.

Buus et al., Science 242:1065 (1988) first described a method for acid elution of bound peptides from MHC. Subsequently, Rammensee and his coworkers (Falk et al., Nature 351:290 (1991) have developed an approach to characterize naturally processed peptides bound to class I molecules. Other investigators have successfully achieved direct amino acid sequencing of the more abundant peptides in various HPLC fractions by conventional automated sequencing of peptides eluted from class I molecules of the B type (Jardetzky, et al., Nature 353:326 (1991) and of the A2.1 type by mass spectrometry (Hunt, et al., Science 225:1261 (1992). A review of the characterization of naturally processed peptides in MHC Class I has been presented by Rötzschke and Falk (Rötzschke and Falk, Immunol. Today 12:447 (1991).

Sette et al., Proc. Natl. Acad. Sci. USA 86:3296 (1989) showed that MHC allele specific motifs could be used to predict MHC binding capacity. Schaeffer et al., Proc. Natl. Acad. Sci. USA 86:4649 (1989) showed that MHC binding was related to immunogenicity. Several authors (De Bruijn et al., Eur. J. Immunol., 21:2963-2970 (1991); Pamer et al., 991 Nature 353:852-955 (1991)) have provided preliminary evidence that class I binding motifs can be applied to the identification of potential immunogenic peptides in animal models. Class I motifs specific for a number of human alleles of a given class I isotype have yet to be described. It is desirable that the combined frequencies of these different alleles should be high enough to cover a large fraction or perhaps the majority of the human outbred population.

Despite the developments in the art, the prior art has yet to provide a useful human peptide-based vaccine or therapeutic agent based on this work. The present invention provides these and other advantages.

### SUMMARY OF THE INVENTION

The present invention provides compositions comprising immunogenic peptides having binding motifs for HLA-A2.1 molecules. The immunogenic peptides, which bind to the appropriate MHC allele, are preferably 9 to 10 residues in length and comprise conserved residues at certain positions such as positions 2 and 9. Moreover, the peptides do not comprise negative binding residues as defined herein at other positions such as positions 1, 3, 6 and/or 7 in the case ofpeptides 9 amino acids in length and positions 1, 3, 4, 5, 7, 8 and/or 9 in the case of peptides 10 amino acids in length. The present invention defines positions within a motif enabling the selection of peptides which will bind efficiently to HLA A2.1.

Epitopes on a number of immunogenic target proteins can be identified using the peptides of the invention. Examples of suitable antigens include prostate cancer specific antigen (PSA), hepatitis B core and surface antigens (HBVc, HBVs) hepatitis C antigens, Epstein-Barr virus antigens, human immunodeficiency type-1 virus (HIV1), Kaposi's sarcoma herpes virus (KSHV), human papilloma virus (HPV) antigens, Lassa virus, mycobacterium tuberculosis (MT), p53, CEA, trypanosome surface antigen (TSA) and Her2/neu. The peptides are thus useful in pharmaceutical compositions for both in vivo and ex vivo therapeutic and diagnostic applications.

### Definitions

The term "peptide" is used interchangeably with "oligopeptide" in the present specification to designate a series of residues, typically L-amino acids, connected one to the other typically by peptide bonds between the alpha-amino and carbonyl groups of adjacent amino acids. The oligopeptides of the invention are less than about 15 residues in length and usually consist of between about 8 and about 11 residues, preferably 9 or 10 residues.

An "immunogenic peptide" is a peptide which comprises an allele-specific motif such that the peptide will bind an MHC molecule and induce a CTL response. Immunogenic peptides of the invention are capable of binding to an appropriate BLA-A2.1 molecule and inducing a cytotoxic T cell response against the antigen from which the immunogenic peptide is derived.

Immunogenic peptides are conveniently identified using the algorithms of the invention. The algorithms are mathematical procedures that produce a score which enables the selection of immunogenic peptides. Typically one uses the algorithmic score with a "binding threshold" to enable selection of peptides that have a high probability of binding at a certain affinity and will in turn be immunogenic. The algorithm is based upon either the effects on MHC binding of a particular amino acid at a particular position of a peptide or the effects on binding of a particular substitution in a motif containing peptide.

A "conserved residue" is an amino acid which occurs in a significantly higher frequency than would be expected by random distribution at a particular position in a peptide. Typically a conserved residue is one where the MHC structure may provide a contact point with the immunogenic peptide. At least one to three or more, preferably two, conserved residues within a peptide of defined length defines a motif for an immunogenic peptide. These residues are typically in close contact with the peptide binding groove, with their side chains buried in specific pockets of the groove itself. Typically, an immunogenic peptide will comprise up to three conserved residues, more usually two conserved residues.

As used herein, "negative binding residues" are amino acids which if present at certain positions (for example, positions 1, 3 and/or 7 of a 9-mer) will result in a peptide being a nonbinder or poor binder and in turn fail to be immunogenic i.e. induce a CTL response.

The term "motif" refers to the pattern of residues in a peptide of defined length, usually about 8 to about 11 amino acids, which is recognized by a particular MHC allele. The peptide motifs are typically different for each human MHC allele and differ in the pattern of the highly conserved residues and negative residues.

The binding motif for an allele can be defined with increasing degrees of precision. In one case, all of the conserved residues are present in the correct positions in a peptide and there are no negative residues in positions 1,3 and/or 7.

The phrases "isolated" or. "biologically pure" refer to material which is substantially or essentially free from components which normally accompany it as found in its native state. Thus, the peptides of this invention do not contain materials normally associated with their in situ environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

### DESCRIPTION OF THE PREFERRED EMBODIMENTS

The present invention relates to the determination of hllele-specific peptide motifs for human Class I MHC (sometimes referred to as HLA) allele subtypes, in particular, peptide motifs recognized by HLA-A2:1 alleles. These motifs are then used to define T cell epitopes from any desired antigen, particularly those associated with human viral diseases, cancers or autoimmune diseases, for which the amino acid sequence of the potential antigen or autoantigen targets is known.

Epitopes on a number of potential target proteins can be identified in this manner. Examples of suitable antigens include prostate specific antigen (PSA), hepatitis B core and surface antigens (HBVc, HBVs) hepatitis C antigens, Epstein-Barr virus antigens, melanoma antigens (e.g., MAGE-1), human immunodeficiency virus (HIV) antigens, human papilloma virus (HPV) antigens, Lassa virus, mycobacterium tuberculosis (MT), p53, CEA, trypanosome surface antigen (TSA) and Her2/neu.

Peptides comprising the epitopes from these antigens are synthesized and then tested for their ability to bind to the appropriate MHC molecules in assays using, for example, purified class I molecules and radioiodonated peptides and/or cells expressing empty class I molecules by, for instance, immnunofluorescent staining and flow microfluorometry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary in vitro or in vivo CTL responses that can give rise to CTL populations capable of reacting with virally infected target cells or tunior cells as potential therapeutic agents.

The MHC class I antigens are encoded by the HLA-A, B, and C loci. HLA-A and B antigens are expressed at the cell surface at approximately equal densities, whereas the expression of HLA-C is sigriificantly lower (perhaps as much as 10-fold lower). Each of these loci have a number of alleles. The peptide binding motifs of the invention are relatively specific for each allelic subtype.

For peptide-based vaccines, the peptides of the present invention preferably comprise a motif recognized by an MHC I molecule having a wide distribution in the human population. Since the MHC alleles occur at different frequencies within different ethnic groups and races, the choice of target MHC allele may depend upon the target population. Table 1 shows the frequency of various alleles at the HLA-A locus products among different races. For instance, the majority of the Caucasoid population can be covered by peptides which bind to four HLA-A allele subtypes, specifically HLA-A2.1, A1, A3.2, and A24.1. Similarly, the majority of the Asian population is encompassed with the addition of peptides binding to a fifth allele HLA-A11.2.

**TABLE 1**

| A Allele/Subtype | N(69)* | A(54) | C(502) |
|---|---|---|---|
| A1 | 10.1(7) | 1.8(1) | 27.4(138) |
| A2.1 | 11.5(8) | 37.0(20) | 39.8(199) |
| A2.2 | 10.1(7) | 0 | 3.3(17) |
| A2.3 | 1.4(1) | 5.5(3) | 0.8(4) |
| A2.4 | - | - | - |
| A2.5 | - | - | - |
| A3.1 | 1.4(1) | 0 | 0.2(0) |
| A3.2 | 5.7(4) | 5.5(3) | 21.5(108) |
| A11.1 | 0 | 5.5(3) | 0 |
| A11.2 | 5.7(4) | 31.4(17) | 8.7(44) |
| A11.3 | 0 | 3.7(2) | 0 |
| A23 | 4.3(3) | - | 3.9(20) |
| A24 | 2.9(2) | 27.7(15) | 15.3(77) |
| A24.2 | - | - | - |
| A24.3 | - | - | - |
| A25 | 1.4(1) | - | 6.9(35) |
| A26.1 | 4.3(3) | 9.2(5) | 5.9(30) |
| A26.2 | 7.2(5) | - | 1.0(5) |
| A26V | - | 3.7(2) | - |
| A28.1 | 10.1(7) | - | 1.6(8) |
| A28.2 | 1.4(1) | - | 7.5(38) |
| A29.1 | 1.4(1) | - | 1.4(7) |
| A29.2 | 10.1(7) | 1.8(1) | 5.3(27) |
| A30.1 | 8.6(6) | - | 4.9(25) |
| A30.2 | 1.4(1) | - | 0.2(1) |
| A30.3 | 7.2(5) | - | 3.9(20) |
| A31 | 4.3(3) | 7.4(4) | 6.9(35) |
| A32 | 2.8(2) | - | 7.1(36) |
| Aw33.1 | 8.6(6) | - | 2.5(13) |
| Aw33.2 | 2.8(2) | 16.6(9) | 1.2(6) |
| Aw34.1 | 1.4(1) | - | - |
| Aw34.2 | 14.5(10) | - | 0.8(4) |
| Aw36 | 5.9(4) | - | - |

| | | | |
|---|---|---|---|
| Table compiled from B. DuPont, Immunobiology of HLA, Vol. I, Histocompatibility Testing 1987, Springer-Verlag, New York 1989. | | | |
| * N - negroid; A = Asian; C = caucasoid. Numbers in parenthesis represent the number of individuals included in the analysis. | | | |

The nomenclature used to describe peptide compounds follows the conventional practice wherein the amino group is presented to the left (the N-terminus) and the carboxyl group to the right (the C-terminus) of each amino acid residue. In the formulae representing selected specific embodiments of the present invention, the amino-and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

The procedures used to identify peptides of the present invention generally follow the methods disclosed in Falk et al., Nature 351:290 (1991), which is incorporated herein by reference. Briefly, the methods involve large-scale isolation of MHC class I molecules, typically by immunoprecipitation or affinity chromatography, from the appropriate cell or cell line. Examples of other methods for isolation of the desired MHC molecule equally well known to the artisan include ion exchange chromatography, lectin chromatography, size exclusion, high performance ligand chromatography, and a combination of all of the above techniques.

In the typical case, immunoprecipitation is used to isolate the desired allele. A number of protocols can be used, depending upon the specificity of the antibodies used. For example, allele-specific mAb reagents can be used for the affinity purification of the HLA-A, HLA-B₁, and HLA-C molecules. Several mAb reagents for the isolation of HLA-A molecules are available. The monoclonal BB7.2 is suitable for isolating HLA-A2 molecules. Affinity columns prepared with these mAbs using standard techniques are successfully used to purify the respective HLA-A allele products.

In addition to allele-specific mAbs, broadly reactive anti-HLA-A, B, C mAbs, such as W6/32 and B9.12.1, and one anti-HLA-B, C mAb, B1.23.2, could be used in altemative affinity purification protocols as described in previous applications.

The peptides bound to the peptide binding groove of the isolated MHC molecules are eluted typically using acid treatment Peptides can also be dissociated from class I molecules by a variety of standard denaturing means, such as heat, pH, detergents, salts, chaotropic agents, or a combination thereof.

Peptide fractions are further separated from the MHC molecules by reversed-phase high performance liquid chromatography (HPLC) and sequenced. Peptides can be separated by a variety of other standard means well known to the artisan, including filtration, ultrafiltration, electrophoresis, size chromatography, precipitation with specific antibodies, ion exchange chromatography, isoelectrofocusing, and the like.

Sequencing of the isolated peptides can be performed according to standard techniques such as Edman degradation (Hunkapiller, M.W., et al., Methods Enzymol. 91, 399 [1983]). Other methods suitable for sequencing include mass spectrometry sequencing of individual peptides as previously described (Hunt, et al., Science 225:1261 (1992), which is incorporated herein by reference). Amino acid sequencing of bulk heterogenous peptides (e.g., pooled HPLC fractions) from different class I molecules typically reveals a characteristic sequence motif for each class I allele.

Definition of motifs specific for different class I alleles allows the identification of potential peptide epitopes from an antigenic protein whose amino acid sequence is known. Typically, identification of potential peptide epitopes is initially carried out using a computer to scan the amino acid sequence of a desired antigen for the presence of motifs. The epitopic sequences are then synthesized. The capacity to bind MHC Class molecules is measured in a variety of different ways. One means is a Class I molecule binding assay as described in the related applications, noted above. Other alternatives described in the literature include inhibition of antigen presentation (Sette, et al., J. Immunol. 141:3893 (1991), in vitro assembly assays (Townsend, et al., Cell 62:285 (1990), and FACS based assays using mutated ells, such as RMA.S (Melief, et al., Eur. J. Immunol. 21:2963 (1991)).

Next, peptides that test positive in the MEC class I binding assay are assayed for the ability of the peptides to induce specific CTL responses in vitro. For instance, Antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. J. Immunol. 18:219 [1988]).

Alternatively, mutant mammalian cell lines that are deficient in their ability to load class I molecules with internally processed peptides, such as the mouse cell lines RMA-S (Kärre, et al., Nature, 319:675 (1986); Ljunggren, et al., Eur. J. Immunol. 21:2963-2970 (1991)), and the human somatic T cell hybrid, T-2 (Cerundolo, et al., Nature 345:449-452 (1990)) and which have been transfected with the appropriate human class I genes are conveniently used, when peptide is added to them, to test for the capacity of the peptide to induce in vitro primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (see Schneider J. Embryol. Exp. Morphol. 27:353-365 [1927]).

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations in vitro for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL is determined by testing against different peptide target cells expressing appropriate or inappropriate human MHC class I. The peptides that test positive in the MHC binding assays and give rise to specific CTL responses are referred to herein as immunogenic peptides.

The immunogenic peptides can be prepared synthetically, or by recombinant DNA technology or from natural sources such as whole viruses or tumors. Although the peptide will preferably be substantially free of other naturally occurring host cell proteins and fragments thereof, in some embodiments the peptides can be synthetically conjugated to native fragments or particles.

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. When possible, it may be desirable to optimize peptides of the invention to a length of 9 or 10 amino acid residues, commensurate in size with endogenously processed viral peptides or tumor cell peptides that are bound to MHC class I molecules on the cell surface.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany and Merrifield, The Peptides, Gross and Meienhofer, eds. (N.Y., Academic Press), pp. 1-284 (1979); and Stewart and Young, Solid Phase Peptide Synthesis, (Rockford, Ill., Pierce), 2d Ed. (1984), incorporated by reference herein.

The peptides can also be modified by extending or decreasing the compound's amino acid sequence, e.g., by the addition or deletion of amino acids. The peptides or analogs of the invention can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well, such as β-γ-δ-amino acids, as well as many derivatives of L-α-amino acids.

Typically, a series of peptides with single amino acid substitutions are employed to determine the effect of electrostatic charge, hydrophobicity, etc. on binding. For instance, a series of positively charged (e.g., Lys or Arg) or negatively charged (e.g., Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place. Such substitutions generally are made in accordance with the following Table 2 when it is desired to finely modulate the characteristics of the peptide.

**TABLE 2**

| Original Residue | Exemplary Substitution |
|---|---|
| Ala | Ser |
| Arg | Lys, His |
| Asn | Gln |
| Asp | Glu |
| Cys | Ser |
| Gln | Asn |
| Glu | Asp |
| Gly | Pro |
| His | Lys; Arg |
| Ile | Leu; Val |
| Leu | Ile; Val |
| Lys | Arg; His |
| Met | Leu; Ile |
| Phe | Tyr; Trp |
| Ser | Thr |
| Thr | Ser |
| Trp | Tyr; Phe |
| Tyr | Trp; Phe |
| Val | Ile; Leu |
| Pro | Gly |

Substantial changes in function (e.g., affinity for MHC molecules or T cell receptors) are made by selecting substitutions that are less conservative than those in Table 2, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, e.g. leucyl, isoleucyl, phenylalanyl, valyl or alanyl; (b) a residue having an electropositive side chain, e.g., lysl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, e.g., glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nibogen, the α-carbon, amide carbonyl, complete replacement of the amide bond, extensions, deletions or backbone crosslinks. See, generally, Spatola, Chemistry and Biochemistry of Amino Acids, peptides and Proteins, Vol. VII (Weinstein ed., 1983).

Modifications of peptides with various amino acid mimetics or unnatural amino acids are particularly useful in increasing the stability of the peptide in vivo. Stability can be assayed in a number of ways. For instance, peptidases and various biological media, such as human plasma and serum, have been used to test stability. See, e.g., Verhoef et al., Eur. J. Drug Metab. Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC. using stability-specific chromatography conditions.

The peptides of the present invention or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated. Exemplary T helperpeptides include tetanus toxoid 830-843, influenza 307-319, malaria circumsporozoite 382-398 and 378-389.

In some embodiments it may be desirable to include in the pharmaceutical compositions of the invention at least one component which primes CTL. Lipids have been identified as agents capable of priming CTL in vivo against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-Serine (P₃CSS) can be used to prime virus specific CTL when covalently attached to an appropriate peptide. See, Deres et al., Nature 342:561-564 (1989), incorporated herein by reference. Peptides of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH₂ acyladon, e.g., by alkanoyl (C₁-C₂₀) or thioglycolyl acetylation, terminal-carboxyl amidation, e.g., ammonia, methylamine, etc. In some instances these modifications may provide sites for linking to a support or other molecule.

The peptides of the invention can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co. (1984), supra.

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A Laboratory Manual, Cold Spring Harbor Press, Cold Spring Harbor, New York (1982), which is incorporated herein by reference. Thus, fusion proteins which comprise one or more peptide sequences of the invention can be used to present the appropriate T cell epitope.

As the coding sequence for peptides of the length contemplated herein can be synthesized by chemical techniques, for example, the phosphotriester method of Matteucci et al., J. Am. Chem. Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

The peptides of the present invention and pharmaceutical and vaccine compositions thereof are useful for administration to mammals, particularly humans, to treat and/or prevent viral infection and cancer. Examples of diseases which can be treated using the immunogenic peptides of the invention include prostate cancer, hepatitis B, hepatitis C, AIDS, renal carcinoma, cervical carcinoma, lymphoma, CMV and condlyloma acuminatum.

For pharmaceutical compositions, the immunogenic peptides of the invention are administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 5000 µg of peptide for a 70 kg patient, followed by boosting dosages of from about 1.0 µg to about 1000 µg of peptide pursuant to a boosting regimen over weeks to months depending upon the patient's response and condition by measuring specific CTL activity in the patient's blood. It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 5000 µg, preferably about 5 µg to 1000 µg for a 70 kg patient per dose. Immunizing doses followed by boosting doses at established intervals, e.g., from one to four weeks, may be required, possibly for a prolonged period of time to effectively immunize an individual, In the case of chronic infection, administration should continue until at least clinical symptoms or laboratory tests indicate that the viral infection has been eliminated or substantially abated and for a period thereafter.

The pharmaceutical, compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides of the invention may also be administered via liposomes, which serve to target the peptides to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes either filled or decorated with a desired peptide of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, eg., liposome size; acid lability arid stability of the liposomes in the blood stream. A variety of methods are available for preparing liposomes, as described in, e.g., Szoka et al., Ann. Rev Biophys. Bioeng. 9:467 (1980), U.S. Patent Nos. 4,235,871, 4,501,728, 4,837,028, and 5,019,369, incorporated herein by reference.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to inter alia the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

In another aspect the present invention is directed to vaccines which contain as an active ingredient an immunogenically effective amount of an immunogenic peptide as described herein. The peptide(s) may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants of the virus or tumor cells. Useful carriers are well known in the art, and include, e.g., thyroglobulin, albumins such as human serum albumin, tetanus toxoid, polyamino acids such as poly(lysine:glutamic acid), influenza, hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of CTLs specific for the desired-antigen, and the host becomes at least partially immune to later infection, or resistant to developing chronic infection.

Vaccine compositions containing the peptides of the invention are administered to-a patient susceptible to or otherwise at risk of viral infection or cancer to elicit an immune response against the antigen and thus enhance the-patient's own immune response capabilities. Such an amount is defined to be an "immunogenically effective dose." In this use, the precise amounts again depend on the patient's state of health and weight, the mode of administration, the nature of the formulation, etc., but generally range from about 1.0 µg to about 5000 µg per 70 kilogram patient, more commonly from about 10 µg to about 500 µg mg per 70 kg of body weight.

In some instances it may be desirable to combine the peptide vaccines of the invention with vaccines which induce neutralizing antibody responses to the virus of interest, particularly to viral envelope antigens.

For therapeutic or immunization purposes, nucleic acids encoding one or more of the peptides of the invention can also be administered to the patient A number of methods are conveniently used to deliver the nucleic acids to the patient. For instance, the nucleic acid can be delivered directly, as "naked DNA". This approach is described, for instance, in Wolff *et*. *al*., *Science* 247: 1465-1468(1990) as well as U.S. Patent Nos. 5,580,859 and 5,589,466. The nucleic acids can also be administered using ballistic delivery as described, for instance, in U.S. Patent No. 5,204,253. Particles comprised solely of DNA can be administered. Alternatively, DNA can be adhered to particles, such as gold particles. The nucleic acids can also be delivered complexed to cationic compounds, such as cationic lipids. Lipid-mediated gene delivery methods are described, for instance, in WO 96/18372; WO 93/24640; Mannino and Gould-Fogerite (1988) *BioTechniques* 6(7): 682-691; Rose U.S. Pat No. 5,279,833; WO 91/06309; and Felgner *et al*. (1987) *Proc. Natl. Acad. Sci. USA* 84: 7413-7414. The peptides of the invention can also be expressed by attenuated viral hosts, such as vaccinia or fowlpox. This approach involves the use of vaccinia virus as a vector to express nucleotide sequences that encode the peptides of the invention. Upon introduction into an acutely or chronically infected host or into a noninfected host, the recombinant vaccinia virus expresses the immunogenic peptide, and thereby elicits a host CTL response. Vaccinia vectors and methods useful in immunization protocols are described in, e.g., U.S. Patent No. 4,722,848, incorporated herein by reference. Another vector is BCG (Bacille Calmette Guerin). BCG vectors are described in Stover et al. (Nature 351:456-460 (1991)) which is incorporated herein by reference. A wide variety of other vectors useful for therapeutic administration or immunization of the peptides of the invention, e.g., Salmonella typhi vectors and the like, will be apparent to those skilled in the art from the description herein.

A preferred means of administering nucleic acids encoding the peptides of the invention uses minigene constructs encoding multiple epitopes of the invention. To create a DNA sequence encoding the selected CTL epitopes (minigene) for expression inhuman cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte epitopes, aleader (signal) sequence, and an endoplasmic reticulum retention signal. In addition, MHC presentation of CTL epitopes may be improved by including synthetic (e.g. poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. The ends of the oligonucleotides are joined using T4 DNA ligase. This synthetic minigene, encoding the CTL epitope polypeptide, can then cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E. coli* origin of replication; and an *E. coli* selectable marker (e.g. ampicillin or kanamycin resistance). Numerous promoters can be used for this purpose, *e*.*g*., the human cytomegalovirus (hCMV) promoter. *See*, U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA vaccines. These sequences could be included in the vector, outside the minigene coding sequence, if found to enhance immunogenicity.

In some embodiments, a bicistronic expression vector; to allow production of the minigene-encoded epitopes and a second protein included to enhance or decrease immunogenicity can be used. Examples of proteins or polypeptides that could beneficially enhance the immune response if co-expressed include cytokines (e.g., IL2, IL12, GM-CSF), cytokine-inducing molecules (e.g. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immmidsuppressive molecules (e.g. TGF-β) may be beneficial in certain diseases.

Once an expression vector is selected, the minigene is cloned into the polylinker region downstream of the promoter. This plasmid is transformed into an appropriate *E. coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in *E*. *coli*, followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). A variety of methods have been described, and new techniques may become available. As noted above, nucleic acids are conveniently formulated with cationic lipids. In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

Target cell sensifization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by 51Cr release, indicates production of MHC presentation of minigene-ericoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for *in vivo* induction of CTLs.

Antigenic peptides may be used to elicit CTL ex vivo, as well. The resulting CTL, can be used to treat chronic infections (viral or bacterial) or tumors in patients that do not respond to other conventional forms of therapy, or will not respond to a peptide vaccine approach of therapy. Ex vivo CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell).

The peptides may also find use as diagnostic reagents. For example, a peptide of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

The following example is offered by way of illustration, not by way of limitation.

### Example 1

Class I antigen isolation was carried out as described in the related applications, noted above. Naturally processed peptides were then isolated and sequenced as described there. An allele-specific motif and algorithms were determined and quantitative binding assays were carried out.

Using the motifs identified above for HLA-A2.1 allele amino acid sequences from a number of antigens were analyzed for the presence of these motifs. Tables 3-5 provide the results of these searches.

The above examples are provided to illustrate the invention but not to limit its scope. Other variants of the invention will be readily apparent to one of ordinary skill in the art and arc encompassed by the appended claims. All publications, patents, and patent applications cited herein are hereby incorporated by reference.

**Table 3**

| SEQ ID NO | AA | Sequence | Source |
|---|---|---|---|
| 1 | 9 | FLYNRPLSV | TSA-1 641 |
| 2 | 9 | VLLPSLFLL | TSA-1 818 |
| 3 | 9 | LLPSLFLLL | TSA-1 819 |
| 4 | 9 | FVDYNFTIV | TSA-1 514 |
| 5 | 9 | KLFPEVIDL | TSA-1 89 |
| 6 | 10 | FLLLGLWGFA | TSA-1 824 |
| 7 | 10 | VLLPSLFLLL | TSA-1 818 |
| 8 | 10 | LLYSDDALHL | TSA-1 398 |
| 9 | 9 | AIYHPQQFV | MT 32k 178 |
| 10 | 9 | AMKADIQHV | MT 85c 317 |
| 11 | 9 | AMLQDMAIL | MT 65k 285 |
| 12 | 9 | DMWEHAFYL | MT superoxide dismutase 160 |
| 13 | 9 | GLFLTTEAV | MT 65k 509 |
| 14 | 9 | ILFTFLHLA | MT alanine dehydrogenase 92 |
| 15 | 9 | KLAGGVAVI | MT 65k 369 |
| 16 | 9 | LMIGTAAAV | MT 85B 15 |
| 17 | 9 | MLQDMAILT | MT 65k 286 |
| 18 | 9 | RLMIGTAAA | MT 85B 14 |
| 19 | 9 | RLVSGLVGA | MT 32k 25 |
| 20 | 9 | RMPAVTDLV | MT70k318 |
| 21 | 9 | SLLEIGEGV | MT 70k 179 |
| 22 | 9 | VLLLDVTPL | MT 70k 363 |
| 23 | 9 | YTYKWETFL | MT 85c 137 |
| 24 | 9 | ALINDQLIM | Lassa gp 343 |
| 25 | 9 | AMLQLDPNA | Lassa Josiah(NP) 376 |
| 26 | 9 | FVFSTSFYL | Lassa gp 434 |
| 27 | 9 | GLTSAVIDA | Lassa Josiah(NP) 444 |
| 28 | 9 | GLVGLVTFL | Lassa Josiah(GP) |
| 29 | 9 | HSTFHLSI | Lassa gp 136 |
| 30 | 9 | ILAADLEKL | Lassa np 101 |
| 31 | 9 | IMTSYQYLI | Lassa gp 213 |
| 32 | 9 | LIALSVLAV | Lassa Josiah(GP) 23 |
| 33 | 9 | LISIFLHLV | Lassa gp 442 |
| 34 | 9 | LLGTFTWTL | Lassa gp 258 |
| 35 | 9 | LMDCIMFDA | Lassa np 531 |
| 36 | 9 | LTYSQLMTL | Lassa np 365 |
| 37 | 9 | MAWGGSYIA | Lassa gp 194 |
| 38 | 9 | NISGYNFSL | Lassa np 240 |
| 39 | 9 | RLFDFNKQA | Lassa gp 314 |
| 40 | 9 | RLLGTFTWT | Lassa gp 257 |
| 41 | 9 | RLRDLNQAV | Lassa np 66 |
| 42 | 9 | RMAWGGSYI | Lassa gp 193 |
| 43 | 9 | STSFYLISI | Lassa gp 437 |
| 44 | 9 | VLIALSVLA | Lassa Josiah(GP) 22 |
| 45 | 9 | YLISIFLHL | Lassa gp 441 |
| 46 | 10 | ALYLLDGLRA | MT 32k 77 |
| 47 | 10 | AVHDTLFYCV | MT alanine dehydrogenase 291 |
| 48 | 10 | GLFLTTEAVV | MT 65k 509 |
| 49 | 10 | LLAAGVADPV | MT 65k 485 |
| 50 | 10 | LLGSFELTGI | MT 70k 427 |
| 51 | 10 | LLLDVTPLSL | MT 70k 364 |
| 52 | 10 | LMIGTAAAVV | MT 85B 15 |
| 53 | 10 | RIANGMGATV | MT alanine dehydrogenase 187 |
| 54 | 10 | SLLEIGEGVV | MT 70k 179 |
| 55 | 10 | ALMDCIMFDA | Lassa np 530 |
| 56 | 10 | GLVGLVTFLL | Lassa Josiah(GP) 42 |
| 57 | 10 | GLYKQPGVPV | Lassa gp 478 |
| 58 | 10 | GLYNFATCGL | Lassa Josiah(GP) 34 |
| 59 | 10 | LLHGLDFSEV | Lassa np 38 |
| 60 | 10 | LMSIISTFHL | Lassa gp 133 |
| 61 | 10 | NLYDHALMSI | Lassa Josiah(GP) 127 |
| 62 | 10 | RLLGTFTWTL | Lassa gp 257 |
| 63 | 10 | VLIALSVLAV | Lassa Josiah(GP) 22 |
| 64 | 10 | YLISIFLHLV | Lassa gp 441 |
| 65 | 9 | LLDEGKQSL | MT Esat6 28 |
| 66 | 9 | LMTFWNPPV | CEA 24 (a) |
| 67 | 9 | VLYGPDTPV | CEA 589 (a) |
| 68 | 9 | LLTFWNPPV | CEA 24 (a) |
| 69 | 10 | VLYGPDAPTV | CEA 233 (a) |
| 70 | 9 | KLSEYLQLV | MAGE 2 |
| 71 | 11 | LLPENNVLSPL | p53 25-35 |
| 72 | 9 | RMPEAAPPV | p53 65-73 |
| 73 | 11 | GLAPPQHLIRV | p53 187-197 |
| 74 | 10 | NLLGRNSFEV | p53 263-272 |
| 75 | 9 | LLGRNSFEV | p53 264-272 |
| 76 | 9 | SLYKGVYEL | Lassa Josiah (gp) 60 |

The peptides listed in Table 4 were identified as described above and are grouped according to pathogen or antigen from which they were derived.

**Table 4**

| **HBV** | | |
|---|---|---|
| **Sequence** | **Source** | **SEQ ID NO** |
| YMDDVVLGV | POL.538 | 77 |

| **HCV** | | |
|---|---|---|
| **Sequence** | **Source** | **SEQ ID NO** |
| LLFLLLADA | NS1/E2.726 | 78 |
| VLVGGVLAA | NS4. 1666 | 79 |
| HMWNFISGI | NS4.1769 | 80 |
| WMNRLIAFA | NS4.1920 | 81 |

| **HIV1** | | |
|---|---|---|
| **Sequence** | **Source** | **SEQ ID NO** |
| YTAFTIPSI | POL.306 | 82 |
| LTFGWCFKLV | NEF.158 | 83 |
| MASDFNLPPV | POL.764 | 84 |
| CTLNFPISPI | POL.175 | 85 |
| LVGKLNWA | POL.438 | 86 |
| LLQLTVWGI | ENV.731 | 87 |
| LTFGWCFKL | NEF.158 | 88 |
| ALVEICTEM | POL.212 | 89 |
| LVGPTPVNI | POL.156 | 90 |
| AIIRILQQL | VPR.59 | 91 |
| KMIGGIGGFI | POL.125 | 92 |
| MTNNPPIPV | GAG.282 | 93 |
| TLNFPISPI | POL.176 | 94 |
| KAACWWAGI | POL.869 | 95 |
| RAMASDFNL | POL.762 | 96 |
| RILQQLLFI | VPR.62 | 97 |

| Het2/neu | | |
|---|---|---|
| **Sequence** | **Source** | **SEQ ID NO** |
| SIISAWGI | Her2/neu.653 | 98 |
| QLFEDNYALA | Her2/neu.106 | 99 |

| CEA | | |
|---|---|---|
| **Sequence** | **Source** | **SEQ ID NO** |
| YLWWVNNQSL | CEA.354 | 100 |
| IMIGVLVGV | CEA.691 | 101 |
| GIMIGVLVGV | CEA.690 | 102 |
| YLWWVNGQSL | CEA.532 | 103 |
| VLYGPDAPTI | CEA.233 | 104 |
| IMIGVLVGVA | CEA.691 | 105 |
| YLSGANLNL | CEA.605 | 106 |

**Table 5 presents the results of further searches of antigens of interest.**

| Peptide | AA | Sequence | Antigen Protein or Molecule | 1st Position | A*0201 |
|---|---|---|---|---|---|
| 1317.02 | 8 | ALPPVAPV | p53 | 69 | 0.0230 |
| 131711 | 11 | LLPENNVLSPV | p53 | 25 | 0.1300 |
| F136.02 | 9 | SLYNTITVL | HIV gag | 77 | 0.0330 |
| F136.03 | 9 | SLYNTISVL | HIV gag | 77 | 0.0190 |
| F136.04 | 9 | SLYNTVSTL | HIV gag | 77 | 0.0320 |
| 32.0005 | 9 | AIYGRPVSA | KSHV | 508 | 0.0560 |
| 32.0006 | 9 | ALIGTMCGI | KSHV | 237 | 0.1500 |
| 32.0008 | 9 | ATLGTVTLL | KSHV | 8 | 0.0280 |
| 32.0016 | 9 | FIALNLSFI | KSHV | 624 | 0.0640 |
| 32.0017 | 9 | FIQNIDFKA | KSHV | 631 | 0.1400 |
| 32.0019 | 9 | FLNSSNLFT | KSHV | 560 | 0.0780 |
| 32.0021 | 9 | FLYVVCSLA | KSHV | 2 | 1.1000 |
| 32.0022 | 9 | FVAVHVPDV | KSHV | 8 | 0.2600 |
| 32.0027 | 9 | GILGTIIFA | KSHV | 244 | 0.0270 |
| 32.0033 | 9 | HLDFWHHEV | KSHV | 168 | 0.2400 |
| 32.0042 | 9 | TTAFTAPL | KSHV | 342 | 0.1300 |
| 32.0050 | 9 | LLGTWMFSV | KSHV | 53 | 1.5000 |
| 32.0053 | 9 | LMWYELSKI | KSHV | 492 | 0.0670 |
| 32.0060 | 9 | MIIIVIAJI | KSHV | 738 | 0.0150 |
| 32.0066 | 9 | NLLDRLLLI | KSHV | 77 | 0.0290 |
| 32.0073 | 9 | RIFYNILEI | KSHV | 20 | 0.0800 |
| 32.0074 | 9 | RLASSVFDL | KSHV | 649 | 0.0670 |
| 32.0076 | 9 | RLGAIPPLV | KSHV | 24 | 0.0150 |
| 32.0078 | 9 | RLYQASAVM | KSHV | 4 | 0.0180 |
| 32.0081 | 9 | SILGCDVSV | KSHV | 226 | 0.0430 |
| 32.0087 | 9 | SVDFYQFRV | KSHV | 59 | 0.0160 |
| 32.0088 | 9 | SVSDFDLRI | KSHV | 245 | 0.0120 |
| 32.0090 | 9 | TLGTVILLV | KSHV | 9 | 0.0830 |
| 32.0099 | 9 | YLVWQPMSA | KSHV | 398 | 0.0130 |
| 32.0114 | 10 | AAVEQILTSV | KSHV | 237 | 0.0210 |
| 32.0118 | 10 | ALIGTMCGIL | KSHV | 237 | 0.0120 |
| 32.0120 | 10 | ATLGTVILLV | KSHV | 8 | 0.0690 |
| 32.0124 | 10 | FLYVVCSLAV | KSHV | 2 | 0.2400 |
| 32.0127 | 10 | GALPICSFVV | KSHV | 27 | 0.0160 |
| 32,0137 | 10 | KLLGTWMFSV | KSHV | 52 | 1.6000 |
| 32.0148 | 10 | LASILGCDV | KSHV | 223 | 0.0160 |
| 32.0150 | 10 | RLSNRICFWA | KSHV | 164 | 0.0130 |
| 32.0154 | 10 | SLVTGFINFI | KSHV | 720 | 0.0210 |
| 32.0159 | 10 | VLATDVTSFL | KSHV | 149 | 0.0190 |
| 32.0160 | 10 | VLLNGWRWRL | KSHV | 16 | 0.2800 |
| 32.0164 | 10 | YLVWQPMSAI | KSHV | 398 | 0.0230 |
| 34.0006 | 8 | QLAKTCPV | p53 | 136 | 0.0110 |
| 34.0132 | 9 | ALBRWGLLV | HHR2/neu | 5 | 0.0960 |
| 34.0133 | 9 | ALCRWGLLV | HER2/neu | 5 | 0.0360 |
| 34.0134 | 9 | AMCRWGLLV | HER2/neu | 5 | 0.0280 |
| 34.0135 | 9 | KLFGSLAFL | HBR2/neu | 369 | 0.1200 |
| 34.0136 | 9 | KLFGSLAFV | HER2/neu | 369 | 0.0900 |
| 34.0137 | 9 | KMFGSLAFL | HER2/neu | 369 | 0.2000 |
| 34.0138 | 9 | KMFGSLAFV | HER2/neu | 369 | 0.1200 |
| 34.0140 | 9 | KMFBQLAKV | p53 | 132 | 0.0980 |
| 34.0141 | 9 | KMFCQLAKV | p53 | 132 | 0.0400 |
| 34.0199 | 10 | RMPEAAPPVV | p53 | 65 | 0.0340 |
| 34.0201 | 10 | ALNKMFCQLV | p53 | 129 | 0.0240 |
| 35.0031 | 9 | VLLVSLGAI | Flu HEMA | 542 | 0.0170 |
| 35.0034 | 9 | LLTEVETPI | Flu VMT2 | 3 | 0.2100 |
| 35.0036 | 9 | RLIQNSLTI | Flu VNUC | 55 | 0.0300 |
| 35.0040 | 9 | KMNIQFTAV | Flu HEMA | 402 | 0.0330 |
| 35.0046 | 10 | GLFGAIAGFI | Flu HEMA | 345 | 0.0451 |
| 35.0047 | 10 | KLESMGIYQI | Flu HEMA | 521 | 0.0120 |
| 35.0048 | 10 | SLPFQNIHPV | Flu HEMA | 306 | 0.0520 |
| 37.0013 | 8 | ALPPVAPV | p53 | 69 | 0.0500 |
| 37.0015 | 9 | ALNKMFCQV | p53 | 129 | 0.0770 |
| 37.0017 | 9 | KLFCQLAKV | p53 | 132 | 0.0640 |
| 37.0018 | 9 | KLCPVQLWV | p53 | 139 | 0.0440 |
| 37.0019 | 9 | CLTIHYNYV | p53 | 229 | 0.0110 |
| 37.0032 | 10 | ALNKMFCQLV | p53 | 129 | 0.0150 |
| 37.0033 | 10 | VLVPYEPPEV | p53 | 216 | 0.1100 |
| 37.0034 | 10 | RLPEAAPPVV | p53 | 65 | 0.0350 |
| 37.0035 | 10 | LLPPQHLIRV | p53 | 188 | 0.0120 |
| 37.0069 | 11 | ILLEDSSGNLV | p53 | 255 | 0.0590 |
| F124.03 | 10 | KLVALGINAV | HCV NS3 | 1406 | 0.0110 |
| F124.04 | 9 | SLMAFTAAV | HCV NS4 | 1789 | 0.1900 |
| F124.06 | 9 | CINGVCWTY | HCV NS3 | 1073 | 0.0910 |
| P124.08 | 9 | TISGVLWQV | HCV NS3 | 1073 | 0.1400 |
| F124.09 | 9 | SISGVLWQV | HCV NS3 | 1073 | 0.1400 |
| F124.10 | 9 | SLMAFTASV | HCV NS4 | 1789 | 0.1200 |
| F124.11 | 9 | GLRDCTMLV | HCV NS5 | 2727 | 0.0120 |
| F124.12 | 10 | KLVALGVNAV | HCVNS3 | 1406 | 0.0200 |
| F124.14 | 10 | KLSGLGLNAV | HCV NS3 | 1406 | 0.0170 |
| F124.23 | 10 | KLVSLGVNAV | HCV NS3 | 1406 | 0.0150 |
| P127.03 | 10 | LLALLSCLTV | HCV Core | 178 | 0.0240 |
| P127.06 | 9 | LLCPAGHAV | HCV NS3 | 1169 | 0.0140 |
| P127.07 | 10 | KLVALGINAV | HCV NS3 | 1406 | 0.0700 |
| P127.08 | 9 | SLMAFTAAV | HCV NS4 | 1789 | 6.5000 |
| P127.09 | 9 | LLFNILGWV | HCV NS4 | 1807 | 1.7000 |

SEQUENCE LISTING

<110> Grey, Howard M.
      Sette, Alessandro
      Sidney, John
      Southwood, Scott
      Epimmune Inc.

<120> HLA-A2.1 Binding Peptides and Their Uses

<130> 18623-58-9PC

<140> WO PCT/US00/24102
<141> 2000-09-01

<160> 187

<170> FastSEQ for Windows Version 3.0

<210> 1
<211> 9
<212> PRT
<213> Artificial Sequence

<220>
<223> TSA-1 641

<400> 1
Phe Leu Tyr Asn Arg Pro Leu Ser Val
 1               5

<210> 2
<211> 9
<212> PRT
<213> Artificial Sequence

<220>
<223> TSA-1 818

<400> 2
Val Leu Leu Pro Ser Leu Phe Leu Leu
 1               5

<210> 3
<211> 9
<212> PRT
<213> Artificial Sequence

<220>
<223> TSA-1 819

<400> 3
Leu Leu Pro Ser Leu Phe Leu Leu Leu
 1               5

<210> 4
<211> 9
<212> PRT
<213> Artificial Sequence

<220>
<223> TSA-1 514

<400> 4
Phe Val Asp Tyr Asn Phe Thr Ile Val
1               5

<210> 5
<211> 9
<212> PRT
<213> Artificial Sequence

<220>
<223> TSA-1 89

<400> 5
Lys Leu Phe Pro Glu Val Ile Asp Leu
1               5

<210> 6
<211> 10
<212> PRT
<213> Artificial Sequence

<220>
<223> TSA-1 824

<400> 6
Phe Leu Leu Leu Gly Leu Trp Gly Phe Ala
1               5                   10

<210> 7
<211> 10
<212> PRT
<213> Artificial Sequence

<220>
<223> TSA-1 818

<400> 7
Val Leu Leu Pro Ser Leu Phe Leu Leu Leu
1               5                   10

<210> 8
<211> 10
<212> PRT
<213> Artificial Sequence

<220>
<223> TSA-1 398

<400> 8
Leu Leu Tyr Ser Asp Asp Ala Leu His Leu
1               5                   10

<210> 9
<211> 9
<212> PRT
<213> Artificial Sequence

<220>
<223> MT 32k 178

<400> 9
Ala Ile Tyr His Pro Gln Gln Phe Val
1               5

<210> 10
<211> 9
<212> PRT
<213> Artificial Sequence

<220>
<223> MT 85c 317

<400> 10
Ala Met Lys Ala Asp Ile Gln His Val
1               5

<210> 11
<211> 9
<212> PRT
<213> Artificial Sequence

<220>
<223> MT 65k 285

<400> 11
Ala Met Leu Gln Asp Met Ala Ile Leu
1               5

<210> 12
<211> 9
<212> PRT
<213> Artificial Sequence

<220>
<223> MT superoxide dismutase 160

<400> 12
Asp Met Trp Glu His Ala Phe Tyr Leu
1               5

<210> 13
<211> 9
<212> PRT
<213> Artificial Sequence

<220>
<223> MT 65k 509

<400> 13
Gly Leu Phe Leu Thr Thr Glu Ala Val
1               5

<210> 14
<211> 9
<212> PRT
<213> Artificial Sequence

<220>
<223> MT alanine dehydrogenase 92

<400> 14
Ile Leu Phe Thr Phe Leu His Leu Ala
1               5

<210> 15
<211> 9
<212> PRT
<213> Artificial Sequence

<220>
<223> MT 65k 369

<400> 15
Lys Leu Ala Gly Gly Val Ala Val Ile
1               5

<210> 16
<211> 9
<212> PRT
<213> Artificial Sequence

<220>
<223> MT 85B 15

<400> 16
Leu Met Ile Gly Thr Ala Ala Ala Val
1               5

<210> 17
<211> 9
<212> PRT
<213> Artificial Sequence

<220>
<223> MT 65k 286

<400> 17
Met Leu Gln Asp Met Ala Ile Leu Thr
1               5

<210> 18
<211> 9
<212> PRT
<213> Artificial Sequence

<220>
<223> MT 85B 14

<400> 18
Arg Leu Met Ile Gly Thr Ala Ala Ala
1               5

<210> 19
<211> 9
<212> PRT
<213> Artificial Sequence

<220>
<223> MT 32k 25

<400> 19
Arg Leu Val Ser Gly Leu Val Gly Ala
1               5

<210> 20
<211> 9
<212> PRT
<213> Artificial Sequence

<220>
<223> MT 70k 318

<400> 20
Arg Met Pro Ala Val Thr Asp Leu Val
1               5

<210> 21
<211> 9
<212> PRT
<213> Artificial Sequence

<220>
<223> MT 70k 179

<400> 21
Ser Leu Leu Glu Ile Gly Glu Gly Val
1               5

<210> 22
<211> 9
<212> PRT
<213> Artificial Sequence

<220>
<223> MT 70k 363

<400> 22
Val Leu Leu Leu Asp Val Thr Pro Leu
1               5

<210> 23
<211> 9
<212> PRT
<213> Artificial Sequence

<220>
<223> MT 85c 137

<400> 23
Tyr Thr Tyr Lys Trp Glu Thr Phe Leu
1               5

<210> 24
<211> 9
<212> PRT
<213> Artificial Sequence

<220>
<223> Lassa gp 343

<400> 24
Ala Leu Ile Asn Asp Gln Leu Ile Met
1               5

<210> 25
<211> 9
<212> PRT
<213> Artificial Sequence

<220>
<223> Lassa Josiah(NP) 376

<400> 25
Ala Met Leu Gln Leu Asp Pro Asn Ala
1               5

<210> 26
<211> 9
<212> PRT
<213> Artificial Sequence

<220>
<223> Lassa gp 434

<400> 26
Phe Val Phe Ser Thr Ser Phe Tyr Leu
1               5

<210> 27
<211> 9
<212> PRT
<213> Artificial Sequence

<220>
<223> Lassa Josiah(NP) 444

<400> 27
Gly Leu Thr Ser Ala Val Ile Asp Ala
1               5

<210> 28
<211> 9
<212> PRT
<213> Artificial Sequence

<220>
<223> Lassa Josiah(GP)

<400> 28
Gly Leu Val Gly Leu Val Thr Phe Leu
1               5

<210> 29
<211> 9
<212> PRT
<213> Artificial Sequence

<220>
<223> Lassa gp 136

<400> 29
Ile Ile Ser Thr Phe His Leu Ser Ile
1               5

<210> 30
<211> 9
<212> PRT
<213> Artificial Sequence

<220>
<223> Lassa np 101

<400> 30
Ile Leu Ala Ala Asp Leu Glu Lys Leu
1               5

<210> 31
<211> 9
<212> PRT
<213> Artificial Sequence

<220>
<223> Lassa gp 213

<400> 31
Ile Met Thr Ser Tyr Gln Tyr Leu Ile
1               5

<210> 32
<211> 9
<212> PRT
<213> Artificial Sequence

<220>
<223> Lassa Josiah(GP) 23

<400> 32
Leu Ile Ala Leu Ser Val Leu Ala Val
1               5

<210> 33
<211> 9
<212> PRT
<213> Artificial Sequence

<220>
<223> Lassa gp 442

<400> 33
Leu Ile Ser Ile Phe Leu His Leu Val
1               5

<210> 34
<211> 9
<212> PRT
<213> Artificial Sequence

<220>
<223> Lassa gp 258

<400> 34
Leu Leu Gly Thr Phe Thr Trp Thr Leu
1               5

<210> 35
<211> 9
<212> PRT
<213> Artificial Sequence

<220>
<223> Lassa np 531

<400> 35
Leu Met Asp Cys Ile Met Phe Asp Ala
1               5

<210> 36
<211> 9
<212> PRT
<213> Artificial Sequence

<220>
<223> Lassa np 365

<400> 36
Leu Thr Tyr Ser Gln Leu Met Thr Leu
1               5

<210> 37
<211> 9
<212> PRT
<213> Artificial Sequence

<220>
<223> Lassa gp 194

<400> 37
Met Ala Trp Gly Gly Ser Tyr Ile Ala
1               5

<210> 38
<211> 9
<212> PRT
<213> Artificial Sequence

<220>
<223> Lassa np 240

<400> 38
Asn Ile Ser Gly Tyr Asn Phe Ser Leu
1               5

<210> 39
<211> 9
<212> PRT
<213> Artificial Sequence

<220>
<223> Lassa gp 314

<400> 39
Arg Leu Phe Asp Phe Asn Lys Gln Ala
1               5

<210> 40
<211> 9
<212> PRT
<213> Artificial Sequence

<220>
<223> Lassa gp 257

<400> 40
Arg Leu Leu Gly Thr Phe Thr Trp Thr
1               5

<210> 41
<211> 9
<212> PRT
<213> Artificial Sequence

<220>
<223> Lassa np 66

<400> 41
Arg Leu Arg Asp Leu Asn Gln Ala Val
1               5

<210> 42
<211> 9
<212> PRT
<213> Artificial Sequence

<220>
<223> Lassa gp 193

<400> 42
Arg Met Ala Trp Gly Gly Ser Tyr Ile
1               5

<210> 43
<211> 9
<212> PRT
<213> Artificial Sequence

<220>
<223> Lassa gp 437

<400> 43
Ser Thr Ser Phe Tyr Leu Ile Ser Ile
1               5

<210> 44
<211> 9
<212> PRT
<213> Artificial Sequence

<220>
<223> Lassa Josiah(GP) 22

<400> 44
Val Leu Ile Ala Leu Ser Val Leu Ala
1               5

<210> 45
<211> 9
<212> PRT
<213> Artificial Sequence

<220>
<223> Lassa gp 441

<400> 45
Tyr Leu Ile Ser Ile Phe Leu His Leu
1               5

<210> 46
<211> 10
<212> PRT
<213> Artificial Sequence

<220>
<223> MT 32k 77

<400> 46
Ala Leu Tyr Leu Leu Asp Gly Leu Arg Ala
1               5                   10

<210> 47
<211> 10
<212> PRT
<213> Artificial Sequence

<220>
<223> MT alanine dehydrogenase 291

<400> 47
Ala Val His Asp Thr Leu Phe Tyr Cys Val
1               5                   10

<210> 48
<211> 10
<212> PRT
<213> Artificial Sequence

<220>
<223> MT 65k 509

<400> 48
Gly Leu Phe Leu Thr Thr Glu Ala Val Val
1               5                   10

<210> 49
<211> 10
<212> PRT
<213> Artificial Sequence

<220>
<223> MT 65k 485

<400> 49
Leu Leu Ala Ala Gly Val Ala Asp Pro Val
1               5                   10

<210> 50
<211> 10
<212> PRT
<213> Artificial Sequence

<220>
<223> MT 70k 427

<400> 50
Leu Leu Gly Ser Phe Glu Leu Thr Gly Ile
1               5                   10

<210> 51
<211> 10
<212> PRT
<213> Artificial Sequence

<220>
<223> MT 70k 364

<400> 51
Leu Leu Leu Asp Val Thr Pro Leu Ser Leu
1               5                   10

<210> 52
<211> 10
<212> PRT
<213> Artificial Sequence

<220>
<223> MT 85B 15

<400> 52
Leu Met Ile Gly Thr Ala Ala Ala Val Val
1               5                   10

<210> 53
<211> 10
<212> PRT
<213> Artificial Sequence

<220>
<223> MT alanine dehydrogenase 187

<400> 53
Arg Ile Ala Asn Gly Met Gly Ala Thr Val
1               5                   10

<210> 54
<211> 10
<212> PRT
<213> Artificial Sequence

<220>
<223> MT 70k 179

<400> 54
Ser Leu Leu Glu Ile Gly Glu Gly Val Val
1               5                   10

<210> 55
<211> 10
<212> PRT
<213> Artificial Sequence

<220>
<223> Lassa np 530

<400> 55
Ala Leu Met Asp Cys Ile Met Phe Asp Ala
1               5                   10

<210> 56
<211> 10
<212> PRT
<213> Artificial Sequence

<220>
<223> Lassa Josiah(GP) 42

<400> 56
Gly Leu Val Gly Leu Val Thr Phe Leu Leu
1               5                   10

<210> 57
<211> 10
<212> PRT
<213> Artificial Sequence

<220>
<223> Lassa gp 478

<400> 57
Gly Leu Tyr Lys Gln Pro Gly Val Pro Val
1               5                   10

<210> 58
<211> 10
<212> PRT
<213> Artificial Sequence

<220>
<223> Lassa Josiah(GP) 34

<400> 58
Gly Leu Tyr Asn Phe Ala Thr Cys Gly Leu
1               5                   10

<210> 59
<211> 10
<212> PRT
<213> Artificial Sequence

<220>
<223> Lassa np 38

<400> 59
Leu Leu His Gly Leu Asp Phe Ser Glu Val
1               5                   10

<210> 60
<211> 10
<212> PRT
<213> Artificial Sequence

<220>
<223> Lassa gp 133

<400> 60
Leu Met Ser Ile Ile Ser Thr Phe His Leu
1               5                   10

<210> 61
<211> 10
<212> PRT
<213> Artificial Sequence

<220>
<223> Lassa Josiah(GP) 127

<400> 61
Asn Leu Tyr Asp His Ala Leu Met Ser Ile
1               5                   10

<210> 62
<211> 10
<212> PRT
<213> Artificial Sequence

<220>
<223> Lassa gp 257

<400> 62
Arg Leu Leu Gly Thr Phe Thr Trp Thr Leu
1               5                   10

<210> 63
<211> 10
<212> PRT
<213> Artificial Sequence

<220>
<223> Lassa Josiah(GP) 127

<400> 63
Val Leu Ile Ala Leu Ser Val Leu Ala Val
1               5                   10

<210> 64
<211> 10
<212> PRT
<213> Artificial Sequence

<220>
<223> Lassa gp 441

<400> 64
Tyr Leu Ile Ser Ile Phe Leu His Leu Val
1               5                   10

<210> 65
<211> 9
<212> PRT
<213> Artificial Sequence

<220>
<223> MT Esat6 28

<400> 65
Leu Leu Asp Glu Gly Lys Gln Ser Leu
1               5

<210> 66
<211> 9
<212> PRT
<213> Artificial Sequence

<220>
<223> CEA 24 (a)

<400> 66
Leu Met Thr Phe Trp Asn Pro Pro Val
1               5

<210> 67
<211> 9
<212> PRT
<213> Artificial Sequence

<220>
<223> CEA 589 (a)

<400> 67
Val Leu Tyr Gly Pro Asp Thr Pro Val
1               5

<210> 68
<211> 9
<212> PRT
<213> Artificial Sequence

<220>
<223> CEA 24 (a)

<400> 68
Leu Leu Thr Phe Trp Asn Pro Pro Val
1               5

<210> 69
<211> 10
<212> PRT
<213> Artificial Sequence

<220>
<223> CEA 233 (a)

<400> 69
Val Leu Tyr Gly Pro Asp Ala Pro Thr Val
1               5                   10

<210> 70
<211> 9
<212> PRT
<213> Artificial Sequence

<220>
<223> MAGE 2

<400> 70
Lys Leu Ser Glu Tyr Leu Gln Leu Val
1               5

<210> 71
<211> 11
<212> PRT
<213> Artificial Sequence

<220>
<223> p53 25-35

<400> 71
Leu Leu Pro Glu Asn Asn Val Leu Ser Pro Leu
1               5                   10

<210> 72
<211> 9
<212> PRT
<213> Artificial Sequence

<220>
<223> p53 65-73

<400> 72
Arg Met Pro Glu Ala Ala Pro Pro Val
1               5

<210> 73
<211> 11
<212> PRT
<213> Artificial Sequence

<220>
<223> p53 187-197

<400> 73
Gly Leu Ala Pro Pro Gln His Leu Ile Arg Val
1               5                   10

<210> 74
<211> 10
<212> PRT
<213> Artificial Sequence

<220>
<223> p53 263-272

<400> 74
Asn Leu Leu Gly Arg Asn Ser Phe Glu Val
1               5                   10

<210> 75
<211> 9
<212> PRT
<213> Artificial Sequence

<220>
<223> p53 264-272

<400> 75
Leu Leu Gly Arg Asn Ser Phe Glu Val
1               5

<210> 76
<211> 9
<212> PRT
<213> Artificial Sequence

<220>
<223> Lassa Josiah (gp) 60

<400> 76
Ser Leu Tyr Lys Gly Val Tyr Glu Leu
1               5

<210> 77
<211> 9
<212> PRT
<213> Artificial Sequence

<220>
<223> HBV POL.538

<400> 77
Tyr Met Asp Asp Val Val Leu Gly Val
1               5

<210> 78
<211> 9
<212> PRT
<213> Artificial Sequence

<220>
<223> HCV NS1/E2.726

<400> 78
Leu Leu Phe Leu Leu Leu Ala Asp Ala
1               5

<210> 79
<211> 9
<212> PRT
<213> Artificial Sequence

<220>
<223> HCV NS4. 1666

<400> 79
Val Leu Val Gly Gly Val Leu Ala Ala
1               5

<210> 80
<211> 9
<212> PRT
<213> Artificial Sequence

<220>
<223> HCV NS4. 1769

<400> 80
His Met Trp Asn Phe Ile Ser Gly Ile
1               5

<210> 81
<211> 9
<212> PRT
<213> Artificial Sequence

<220>
<223> HCV NS4. 1920

<400> 81
Trp Met Asn Arg Leu Ile Ala Phe Ala
1               5

<210> 82
<211> 9
<212> PRT
<213> Artificial Sequence

<220>
<223> HIV1 POL.306

<400> 82
Tyr Thr Ala Phe Thr Ile Pro Ser Ile
1               5

<210> 83
<211> 10
<212> PRT
<213> Artificial Sequence

<220>
<223> HIV1 NEF.158

<400> 83
Leu Thr Phe Gly Trp Cys Phe Lys Leu Val
1               5                   10

<210> 84
<211> 10
<212> PRT
<213> Artificial Sequence

<220>
<223> HIV1 POL.764

<400> 84
Met Ala Ser Asp Phe Asn Leu Pro Pro Val
1               5                   10

<210> 85
<211> 10
<212> PRT
<213> Artificial Sequence

<220>
<223> HIV POL.175

<400> 85
Cys Thr Leu Asn Phe Pro Ile Ser Pro Ile
1               5                   10

<210> 86
<211> 9
<212> PRT
<213> Artificial Sequence

<220>
<223> HIV1 POL.438

<400> 86
Lys Leu Val Gly Lys Leu Asn Trp Ala
1               5

<210> 87
<211> 9
<212> PRT
<213> Artificial Sequence

<220>
<223> HIV1 ENV.731

<400> 87
Leu Leu Gln Leu Thr Val Trp Gly Ile
1               5

<210> 88
<211> 9
<212> PRT
<213> Artificial Sequence

<220>
<223> HIV1 NEF.158

<400> 88
Leu Thr Phe Gly Trp Cys Phe Lys Leu
1               5

<210> 89
<211> 9
<212> PRT
<213> Artificial Sequence

<220>
<223> HIV1 POL.212

<400> 89
Ala Leu Val Glu Ile Cys Thr Glu Met
1               5

<210> 90
<211> 9
<212> PRT
<213> Artificial Sequence

<220>
<223> HIV1 POL.156

<400> 90
Leu Val Gly Pro Thr Pro Val Asn Ile
1               5

<210> 91
<211> 9
<212> PRT
<213> Artificial Sequence

<220>
<223> HIV1 VPR.59

<400> 91
Ala Ile Ile Arg Ile Leu Gln Gln Leu
1               5

<210> 92
<211> 10
<212> PRT
<213> Artificial Sequence

<220>
<223> HIV1 POL.125

<400> 92
Lys Met Ile Gly Gly Ile Gly Gly Phe Ile
1               5                   10

<210> 93
<211> 9
<212> PRT
<213> Artificial Sequence

<220>
<223> HIV1 GAG.282

<400> 93
Met Thr Asn Asn Pro Pro Ile Pro Val
1               5

<210> 94
<211> 9
<212> PRT
<213> Artificial Sequence

<220>
<223> HIV1 POL.176

<400> 94
Thr Leu Asn Phe Pro Ile Ser Pro Ile
1               5

<210> 95
<211> 9
<212> PRT
<213> Artificial Sequence

<220>
<223> HIV1 POL.869

<400> 95
Lys Ala Ala Cys Trp Trp Ala Gly Ile
1               5

<210> 96
<211> 9
<212> PRT
<213> Artificial Sequence

<220>
<223> HIV1 POL.762

<400> 96
Arg Ala Met Ala Ser Asp Phe Asn Leu
1               5

<210> 97
<211> 9
<212> PRT
<213> Artificial Sequence

<220>
<223> HIV1 VPR.62

<400> 97
Arg Ile Leu Gln Gln Leu Leu Phe Ile
1               5

<210> 98
<211> 9
<212> PRT
<213> Artificial Sequence

<220>
<223> Her2/neu.653

<400> 98
Ser Ile Ile Ser Ala Val Val Gly Ile
1               5

<210> 99
<211> 10
<212> PRT
<213> Artificial Sequence

<220>
<223> Her2/neu.106

<400> 99
Gln Leu Phe Glu Asp Asn Tyr Ala Leu Ala
1               5                   10

<210> 100
<211> 10
<212> PRT
<213> Artificial Sequence

<220>
<223> CEA.354

<400> 100
Tyr Leu Trp Trp Val Asn Asn Gln Ser Leu
1               5                   10

<210> 101
<211> 9
<212> PRT
<213> Artificial Sequence

<220>
<223> CEA.691

<400> 101
Ile Met Ile Gly Val Leu Val Gly Val
1               5

<210> 102
<211> 10
<212> PRT
<213> Artificial Sequence

<220>
<223> CEA.690

<400> 102
Gly Ile Met Ile Gly Val Leu Val Gly Val
1               5                   10

<210> 103
<211> 10
<212> PRT
<213> Artificial Sequence

<220>
<223> CEA.532

<400> 103
Tyr Leu Trp Trp Val Asn Gly Gln Ser Leu
1               5                   10

<210> 104
<211> 10
<212> PRT
<213> Artificial Sequence

<220>
<223> CEA.233

<400> 104
Val Leu Tyr Gly Pro Asp Ala Pro Thr Ile
1               5                   10

<210> 105
<211> 10
<212> PRT
<213> Artificial Sequence

<220>
<223> CEA.691

<400> 105
Ile Met Ile Gly Val Leu Val Gly Val Ala
1               5                   10

<210> 106
<211> 9
<212> PRT
<213> Artificial Sequence

<220>
<223> CEA.605

<400> 106
Tyr Leu Ser Gly Ala Asn Leu Asn Leu
1               5

<210> 107
<211> 8
<212> PRT
<213> Artificial Sequence

<220>
<223> p53 69, peptide 1317.02, peptide 37.0013

<400> 107
Ala Leu Pro Pro Val Ala Pro Val
1               5

<210> 108
<211> 11
<212> PRT
<213> Artificial Sequence

<220>
<223> p53 25, peptide 1317.11

<400> 108
Leu Leu Pro Glu Asn Asn Val Leu Ser Pro Val
1               5                   10

<210> 109
<211> 9
<212> PRT
<213> Artificial Sequence

<220>
<223> HIV gag 77, peptide F136.02

<400> 109
Ser Leu Tyr Asn Thr Ile Thr Val Leu
1               5

<210> 110
<211> 9
<212> PRT
<213> Artificial Sequence

<220>
<223> HIV gag 77, peptide F136.03

<400> 110
Ser Leu Tyr Asn Thr Ile Ser Val Leu
1               5

<210> 111
<211> 9
<212> PRT
<213> Artificial Sequence

<220>
<223> HIV gag 77, peptide F136.04

<400> 111
Ser Leu Tyr Asn Thr Val Ser Thr Leu
1               5

<210> 112
<211> 9
<212> PRT
<213> Artificial Sequence

<220>
<223> KSHV 508, peptide 32.0005

<400> 112
Ala Ile Tyr Gly Arg Pro Val Ser Ala
1               5

<210> 113
<211> 9
<212> PRT
<213> Artificial Sequence

<220>
<223> KSHV 237, peptide 32.0006

<400> 113
Ala Leu Ile Gly Thr Met Cys Gly Ile
1               5

<210> 114
<211> 9
<212> PRT
<213> Artificial Sequence

<220>
<223> KSHV 8, peptide 32.0008

<400> 114
Ala Thr Leu Gly Thr Val Ile Leu Leu
1               5

<210> 115
<211> 9
<212> PRT
<213> Artificial Sequence

<220>
<223> KSHV 624, peptide 32.0016

<400> 115
Phe Ile Ala Leu Asn Leu Ser Phe Ile
1               5

<210> 116
<211> 9
<212> PRT
<213> Artificial Sequence

<220>
<223> KSHV 631, peptide 32.0017

<400> 116
Phe Ile Gln Asn Ile Asp Phe Lys Ala
1               5

<210> 117
<211> 9
<212> PRT
<213> Artificial Sequence

<220>
<223> KSHV 560, peptide 32.0019

<400> 117
Phe Leu Asn Ser Ser Asn Leu Phe Thr
1               5

<210> 118
<211> 9
<212> PRT
<213> Artificial Sequence

<220>
<223> KSHV 2, peptide 32.0021

<400> 118
Phe Leu Tyr Val Val Cys Ser Leu Ala
1               5

<210> 119
<211> 9
<212> PRT
<213> Artificial Sequence

<220>
<223> KSHV 8, peptide 32.0022

<400> 119
Phe Val Ala Val His Val Pro Asp Val
1               5

<210> 120
<211> 9
<212> PRT
<213> Artificial Sequence

<220>
<223> KSHV 244, peptide 32.0027

<400> 120
Gly Ile Leu Gly Thr Ile Ile Phe Ala
1               5

<210> 121
<211> 9
<212> PRT
<213> Artificial Sequence

<220>
<223> KSHV 168, peptide 32.0033

<400> 121
His Leu Asp Phe Trp His His Glu Val
1               5

<210> 122
<211> 9
<212> PRT
<213> Artificial Sequence

<220>
<223> KSHV 342, peptide 32.0042

<400> 122
Ile Thr Ala Thr Phe Thr Ala Pro Leu
1               5

<210> 123
<211> 9
<212> PRT
<213> Artificial Sequence

<220>
<223> KSHV 53, peptide 32.0050

<400> 123
Leu Leu Gly Thr Trp Met Phe Ser Val
1               5

<210> 124
<211> 9
<212> PRT
<213> Artificial Sequence

<220>
<223> KSHV 492, peptide 32.0053

<400> 124
Leu Met Trp Tyr Glu Leu Ser Lys Ile
1               5

<210> 125
<211> 9
<212> PRT
<213> Artificial Sequence

<220>
<223> KSHV 738, peptide 32.0060

<400> 125
Met Ile Ile Ile Val Ile Ala Ile Ile
1               5

<210> 126
<211> 9
<212> PRT
<213> Artificial Sequence

<220>
<223> KSHV 77, peptide 32.0066

<400> 126
Asn Leu Leu Asp Arg Leu Leu Leu Ile
1               5

<210> 127
<211> 9
<212> PRT
<213> Artificial Sequence

<220>
<223> KSHV 20, peptide 32.0073

<400> 127
Arg Ile Phe Tyr Asn Ile Leu Glu Ile
1               5

<210> 128
<211> 9
<212> PRT
<213> Artificial Sequence

<220>
<223> KSHV 649, peptide 32.0074

<400> 128
Arg Leu Ala Ser Ser Val Phe Asp Leu
1               5

<210> 129
<211> 9
<212> PRT
<213> Artificial Sequence

<220>
<223> KSHV 24, peptide 32.0076

<400> 129
Arg Leu Gly Ala Ile Pro Pro Leu Val
1               5

<210> 130
<211> 9
<212> PRT
<213> Artificial Sequence

<220>
<223> KSHV 4, peptide 32.0078

<400> 130
Arg Leu Tyr Gln Ala Ser Ala Val Met
1               5

<210> 131
<211> 9
<212> PRT
<213> Artificial Sequence

<220>
<223> KSHV 226, peptide 32.0081

<400> 131
Ser Ile Leu Gly Cys Asp Val Ser Val
1               5

<210> 132
<211> 9
<212> PRT
<213> Artificial Sequence

<220>
<223> KSHV 59, peptide 32.0087

<400> 132
Ser Val Asp Phe Tyr Gln Phe Arg Val
1               5

<210> 133
<211> 9
<212> PRT
<213> Artificial Sequence

<220>
<223> KSHV 245, peptide 32.0088

<400> 133
Ser Val Ser Asp Phe Asp Leu Arg Ile
1               5

<210> 134
<211> 9
<212> PRT
<213> Artificial Sequence

<223> KSHV 9, peptide 32.0090

<400> 134
Thr Leu Gly Thr Val Ile Leu Leu Val
1               5

<210> 135
<211> 9
<212> PRT
<213> Artificial Sequence

<220>
<223> KSHV 398, peptide 32.0099

<400> 135
Tyr Leu Val Trp Gln Pro Met Ser Ala
1               5

<210> 136
<211> 10
<212> PRT
<213> Artificial Sequence

<220>
<223> KSHV 237, peptide 32.0114

<400> 136
Ala Ala Val Glu Gln Ile Leu Thr Ser Val
1               5                   10

<210> 137
<211> 10
<212> PRT
<213> Artificial Sequence

<220>
<223> KSHV 237, peptide 32.0118

<400> 137
Ala Leu Ile Gly Thr Met Cys Gly Ile Leu
1               5                   10

<210> 138
<211> 10
<212> PRT
<213> Artificial Sequence

<220>
<223> KSVH 8, peptide 32.0120

<400> 138
Ala Thr Leu Gly Thr Val Ile Leu Leu Val
1               5                   10

<210> 139
<211> 10
<212> PRT
<213> Artificial Sequence

<220>
<223> KSHV 2, peptide 32.0124

<400> 139
Phe Leu Tyr Val Val Cys Ser Leu Ala Val
1               5                   10

<210> 140
<211> 10
<212> PRT
<213> Artificial Sequence

<220>
<223> KSHV 27, peptide 32.0127

<400> 140
Gly Ala Leu Pro Ile Cys Ser Phe Val Val
1               5                   10

<210> 141
<211> 10
<212> PRT
<213> Artificial Sequence

<220>
<223> KSHV 52, peptide 32.0137

<400> 141
Lys Leu Leu Gly Thr Trp Met Phe Ser Val
1               5                   10

<210> 142
<211> 10
<212> PRT
<213> Artificial Sequence

<220>
<223> KSHV 223, peptide 32.0148

<400> 142
Gln Leu Ala Ser Ile Leu Gly Cys Asp Val
1               5                   10

<210> 143
<211> 10
<212> PRT
<213> Artificial Sequence

<220>
<223> KSHV 164, peptide 32.0150

<400> 143
Arg Leu Ser Asn Arg Ile Cys Phe Trp Ala
1               5                   10

<210> 144
<211> 10
<212> PRT
<213> Artificial Sequence

<220>
<223> KSHV 720, peptide 32.0154

<400> 144
Ser Leu Val Thr Gly Phe Ile Asn Phe Ile
1               5                   10

<210> 145
<211> 10
<212> PRT
<213> Artificial Sequence

<220>
<223> KSHV 149, peptide 32.0159

<400> 145
Val Leu Ala Thr Asp Val Thr Ser Phe Leu
1               5                   10

<210> 146
<211> 10
<212> PRT
<213> Artificial Sequence

<220>
<223> KSHV 16, peptide 32.0160

<400> 146
Val Leu Leu Asn Gly Trp Arg Trp Arg Leu
1               5                   10

<210> 147
<211> 10
<212> PRT
<213> Artificial Sequence

<220>
<223> KSHV 398, peptide 32.0164

<400> 147
Tyr Leu Val Trp Gln Pro Met Ser Ala Ile
1               5                   10

<210> 148
<211> 8
<212> PRT
<213> Artificial Sequence

<220>
<223> p53 136, peptide 34.0006

<400> 148
Gln Leu Ala Lys Thr Cys Pro Val
1               5

<210> 149
<211> 9
<212> PRT
<213> Artificial Sequence

<220>
<223> HER2/neu 5, peptide 34.0132

<400> 149
Ala Leu Asx Arg Trp Gly Leu Leu Val
1               5

<210> 150
<211> 9
<212> PRT
<213> Artificial Sequence

<220>
<223> HER2/neu 5, peptide 34.0133

<400> 150
Ala Leu Cys Arg Trp Gly Leu Leu Val
1               5

<210> 151
<211> 9
<212> PRT
<213> Artificial Sequence

<220>
<223> Her2/neu 5, peptide 34.0134

<400> 151
Ala Met Cys Arg Trp Gly Leu Leu Val
1               5

<210> 152
<211> 9
<212> PRT
<213> Artificial Sequence

<220>
<223> HER2/neu 369, peptide 34.0135

<400> 152
Lys Leu Phe Gly Ser Leu Ala Phe Leu
1               5

<210> 153
<211> 9
<212> PRT
<213> Artificial Sequence

<220>
<223> HER2/neu 369, peptide 34.0136

<400> 153
Lys Leu Phe Gly Ser Leu Ala Phe Val
1               5

<210> 154
<211> 9
<212> PRT
<213> Artificial Sequence

<220>
<223> HER2/neu 369, peptide 34.0137

<400> 154
Lys Met Phe Gly Ser Leu Ala Phe Leu
1               5

<210> 155
<211> 9
<212> PRT
<213> Artificial Sequence

<220>
<223> HER2/neu 369, peptide 34.0138

<400> 155
Lys Met Phe Gly Ser Leu Ala Phe Val
1               5

<210> 156
<211> 9
<212> PRT
<213> Artificial Sequence

<220>
<223> p53 132, peptide 34.0140

<400> 156
Lys Met Phe Asx Gln Leu Ala Lys Val
1               5

<210> 157
<211> 9
<212> PRT
<213> Artificial Sequence

<220>
<223> p53 132, peptide 34.0141

<400> 157
Lys Met Phe Cys Gln Leu Ala Lys Val
1               5

<210> 158
<211> 10
<212> PRT
<213> Artificial Sequence

<220>
<223> p53 65, peptide 34.0199

<400> 158
Arg Met Pro Glu Ala Ala Pro Pro Val Val
1               5                   10

<210> 159
<211> 10
<212> PRT
<213> Artificial Sequence

<220>
<223> p53 129, peptide 34.0201, peptide 37.0032

<400> 159
Ala Leu Asn Lys Met Phe Cys Gln Leu Val
1               5                   10

<210> 160
<211> 9
<212> PRT
<213> Artificial Sequence

<220>
<223> Flu HEMA 542, peptide 35.0031

<400> 160
Val Leu Leu Val Ser Leu Gly Ala Ile
1               5

<210> 161
<211> 9
<212> PRT
<213> Artificial Sequence

<220>
<223> Flu VMT2 3, peptide 35.0034

<400> 161
Leu Leu Thr Glu Val Glu Thr Pro Ile
1               5

<210> 162
<211> 9
<212> PRT
<213> Artificial Sequence

<220>
<223> Flu VNUC 55, peptide 35.0036

<400> 162
Arg Leu Ile Gln Asn Ser Leu Thr Ile
1               5

<210> 163
<211> 9
<212> PRT
<213> Artificial Sequence

<220>
<223> Flu HEMA 402, peptide 35.0040

<400> 163
Lys Met Asn Ile Gln Phe Thr Ala Val
1               5

<210> 164
<211> 10
<212> PRT
<213> Artificial Sequence

<220>
<223> Flu HEMA 345, peptide 35.0046

<400> 164
Gly Leu Phe Gly Ala Ile Ala Gly Phe Ile
1               5                   10

<210> 165
<211> 10
<212> PRT
<213> Artificial Sequence

<220>
<223> Flu HEMA 521, peptide 35.0047

<400> 165
Lys Leu Glu Ser Met Gly Ile Tyr Gln Ile
1               5                   10

<210> 166
<211> 10
<212> PRT
<213> Artificial Sequence

<220>
<223> Flu HEMA 306, peptide 35.0048

<400> 166
Ser Leu Pro Phe Gln Asn Ile His Pro Val
1               5                   10

<210> 167
<211> 9
<212> PRT
<213> Artificial Sequence

<220>
<223> p53 129, peptide 37.0015

<400> 167
Ala Leu Asn Lys Met Phe Cys Gln Val
1               5

<210> 168
<211> 9
<212> PRT
<213> Artificial Sequence

<220>
<223> p53 132, peptide 37.0017

<400> 168
Lys Leu Phe Cys Gln Leu Ala Lys Val
1               5

<210> 169
<211> 9
<212> PRT
<213> Artificial Sequence

<220>
<223> p53 139, peptide 37.0018

<400> 169
Lys Leu Cys Pro Val Gln Leu Trp Val
1               5

<210> 170
<211> 9
<212> PRT
<213> Artificial Sequence

<220>
<223> p53 229, peptide 37.0019

<400> 170
Cys Leu Thr Ile His Tyr Asn Tyr Val
1               5

<210> 171
<211> 10
<212> PRT
<213> Artificial Sequence

<220>
<223> p53 216, peptide 37.0033

<400> 171
Val Leu Val Pro Tyr Glu Pro Pro Glu Val
1               5                   10

<210> 172
<211> 10
<212> PRT
<213> Artificial Sequence

<220>
<223> p53 65, peptide 37.0034

<400> 172
Arg Leu Pro Glu Ala Ala Pro Pro Val Val
1               5                   10

<210> 173
<211> 10
<212> PRT
<213> Artificial Sequence

<220>
<223> p53 188, peptide 37.0035

<400> 173
Leu Leu Pro Pro Gln His Leu Ile Arg Val
1               5                   10

<210> 174
<211> 11
<212> PRT
<213> Artificial Sequence

<220>
<223> p53 255, peptide 37.0069

<400> 174
Ile Leu Leu Glu Asp Ser Ser Gly Asn Leu Val
1               5                   10

<210> 175
<211> 10
<212> PRT
<213> Artificial Sequence

<220>
<223> HCV NS3 1406, peptide F124.03, peptide F127.07

<400> 175
Lys Leu Val Ala Leu Gly Ile Asn Ala Val
1               5                   10

<210> 176
<211> 9
<212> PRT
<213> Artificial Sequence

<220>
<223> HCV NS4 1789, peptide F124.04, peptide F127.08

<400> 176
Ser Leu Met Ala Phe Thr Ala Ala Val
1               5

<210> 177
<211> 9
<212> PRT
<213> Artificial Sequence

<220>
<223> HCV NS3 1073, peptide F124.06

<400> 177
Cys Ile Asn Gly Val Cys Trp Thr Val
1               5

<210> 178
<211> 9
<212> PRT
<213> Artificial Sequence

<220>
<223> HCV NS3 1073, peptide F124.08

<400> 178
Thr Ile Ser Gly Val Leu Trp Gln Val
1               5

<210> 179
<211> 9
<212> PRT
<213> Artificial Sequence

<220>
<223> HCV NS3 1073, peptide F124.09

<400> 179
Ser Ile Ser Gly Val Leu Trp Gln Val
1               5

<210> 180
<211> 9
<212> PRT
<213> Artificial Sequence

<220>
<223> HCV NS4 1789, peptide F124.10

<400> 180
Ser Leu Met Ala Phe Thr Ala Ser Val
1               5

<210> 181
<211> 9
<212> PRT
<213> Artificial Sequence

<220>
<223> HCV NS5 2727, peptide 124.11

<400> 181
Gly Leu Arg Asp Cys Thr Met Leu Val
1               5

<210> 182
<211> 10
<212> PRT
<213> Artificial Sequence

<220>
<223> HCV NS3 1406, peptide F124.12

<400> 182
Lys Leu Val Ala Leu Gly Val Asn Ala Val
1               5                   10

<210> 183
<211> 10
<212> PRT
<213> Artificial Sequence

<220>
<223> HCV NS3 1406, peptide F124.14

<400> 183
Lys Leu Ser Gly Leu Gly Leu Asn Ala Val
1               5                   10

<210> 184
<211> 10
<212> PRT
<213> Artificial Sequence

<220>
<223> HCV NS3 1406, peptide F124.23

<400> 184
Lys Leu Val Ser Leu Gly Val Asn Ala Val
1               5                   10

<210> 185
<211> 10
<212> PRT
<213> Artificial Sequence

<220>
<223> HCV Core 178, peptide F127.03

<400> 185
Leu Leu Ala Leu Leu Ser Cys Leu Thr Val
1               5                   10

<210> 186
<211> 9
<212> PRT
<213> Artificial Sequence

<220>
<223> HCV NS3 1169, peptide F127.06

<400> 186
Leu Leu Cys Pro Ala Gly His Ala Val
1               5

<210> 187
<211> 9
<212> PRT
<213> Artificial Sequence

<220>
<223> HCV NS4 1807, peptide F127.09

<400> 187
Leu Leu Phe Asn Ile Leu Gly Trp Val
1               5

### Exemplary support in the specification

| | |
|---|---|
| 1 | page 3, lines 13-17; Table 5 at pages 29-30; page 10, lines 3-13 |
| 2 | page 15, lines 11-13 |
| 3 | page 18, lines 20-26 |
| 4 | page 13, lines 4-8 |
| 5 | page 13, lines 22-27 |
| 6 | page 17, lines 13-29 |
| 7 | page 13, lines 9-15 |
| 8 | page 16, lines 26-32 |
| 9 | page 22, lines 26-29 |
| 10 | page 19, lines 16-17 |
| 11 | page 20, lines 6-17 |
| 12 | page 20, lines 7-8 |
| 13 | page 20, lines 13-14 |
| 14 | page 21, lines 5-9 |
| 15 | page 21, lines 29-32 |
| 16 | page 21, lines 32-33 |
| 17 | page 21, lines 33-34 |
| 18 | page 21, line 29 through page 22, line 2 |
| 19 | page 15, lines 11-15 |
| 20 | page 22, lines 23-32 |
| 21 | page 19, line 16 through page 20, line 5 |
| 22 | page 22, line 33 through page 23, line 4 |

## Claims

1. An isolated HCV peptide of 15 residues or less, comprising a sequence selected from the group consisting of: KLVALGINAV (SEQ ID NO:175) SLMAFTAAV (SEQ ID NO:176) CINGVCWTV (SEQ ID NO:177) TISGVLWQV (SEQ ID NO:178) SISGVLWQV (SEQ ID NO:179) SLMAFTASV(SEQ ID NO:180) GLRDCTMLV (SEQ ID NO:181) KLVALGVNAV(SEQ ID NO:182) KLSGLGLNAV (SEQ ID NO:183) LLCPAGHAV (SEQ ID NO:186) LLFNILGWV (SEQ ID NO:187) and
any of the above sequences modified by one or two conservative substitutions.

2. A composition comprising the peptide of claim 1.

3. The composition of claim 2, further comprising a CTL-inducing peptide.

4. The composition of claim 2, further comprising a T helper peptide.

5. The composition of any one of claims 2-4, further comprising a lipid.

6. The composition of any one of claims 2-4, further comprising a liposome.

7. The composition of claim 2, wherein said peptide is linked to a spacer molecule.

8. The composition of any one of claims 2-4, further comprising a carrier.

9. The composition of any one of claims 2-4, further comprising an antigen presenting cell.

10. An isolated nucleic acid encoding the peptide of claim 1.

11. An isolated nucleic acid encoding the peptide of claim 1 and at least one additional peptide or signal sequence.

12. The isolated nucleic acid of claim 11, wherein said at least one additional peptide or signal sequence is a CTL-inducing peptide.

13. The isolated nucleic acid of claim 11, wherein said at least one additional peptide or signal sequence is a T helper peptide.

14. An isolated nucleic acid encoding the peptide of claim 1 and at least one protein or polypeptide included to enhance or decrease immunogenicity.

15. A composition comprising the nucleic acid of any one of claims 10-14.

16. The composition of claim 15, further comprising a lipid.

17. The composition of claim 15, further comprising a liposome.

18. The composition of claim 15, further comprising a carrier.

19. The use of the peptide of claim 1 in the manufacture of a medicament for the prevention or treatment of HCV.

20. The use according to 19, wherein CTL cells are generated ex vivo using said peptide in combination with a patient's CTL precursor cells.

21. The use of the nucleic acid of any of claims 10-14 in the manufacture of a medicament for the prevention or treatment of HCV.

22. The use of the peptide of claim 1 as a diagnostic reagent to determine treatment, prognosis, or risk of an HCV infection.

23. A composition comprising an immunogenic peptide having an HLA-A2.1 binding motif, which immunogenic peptide is selected from a group consisting of SEQ ID Nos: 1 through 187.

24. The composition of claim 23 wherein the immunogenic peptide has a sequence from hepatitis B virus and is SEQ ID NO:77.

25. The composition of claim 23 wherein the immunogenic peptide has a sequence from hepatis C virus and is selected from the group consisting of SEQ ID Nos: 78-81.

26. The composition of claim 23 wherein the immunogenic peptide has a sequence from human immunodeficiency virus and is selected from the group consisting of SEQ ID Nos: 82-97.

27. The composition of claim 23 wherein the immunogenic peptide has a sequence from Her2/neu and is selected from the group consisting of SEQ ID Nos: 98-99.

28. The composition of claim 23 wherein the immunogenic peptide has a sequence from CEA and is selected from the group consisting of SEQ ID Nos: 100-106.

29. The composition of claim 23 wherein the immunogenic peptide has a sequence from p53 and is selected from the group consisting of SEQ ID Nos: 71-74.

30. The composition of claim 23 wherein the immunogenic peptide has a sequence from MAGE and is SEQ ID N0:70.

31. The composition of claim 23 further comprising a pharmaceutically acceptable excipient

32. The composition of claim 23 wherein the peptide is admixed or linked to a second molecule.

33. The composition of claim 23 further comprising a liposome.

34. The composition of claim 23 wherein the peptide is complexed with an HLA-A2.1 molecule that is present on an antigen-presenting cell.

35. An recombinant nucleic acid sequence encoding an immunogenic peptide having an HLA-A2.1 binding motif, which immunogenic peptide is selected from a group consisting of SEQ ID Nos: 1 through 187.

36. A method of inducing a cytotoxic T cell response against a preselected antigen in a patient expressing an HLA-A2.1 molecule, the method comprising contacting cytotoxic T cells from the patient with a composition comprising an immunogenic peptide selected from the group consisting of SEQ ID Nos: 1 through 187.
